# EUROPÄISCHE PATENTSCHRIFT

(11) **EP 4 646 489 B1**
(45) Veröffentlichungstag und Bekanntmachung des Hinweises auf die Patenterteilung: **28.01.2026**
(21) Anmeldenummer: 24716681.2
(22) Anmeldetag: 27.03.2024
(51) Int. Cl.: C12Q 1/04, G01N 1/30

(54) **GRAM-FÄRBEMETHODE**
GRAM STAINING METHOD
PROCÉDÉ DE COLORATION DE GRAM

(43) Veröffentlichungstag der Anmeldung: 12.11.2025
(73) Patentinhaber: Bio-Gram Microbiology GmbH, 67550 Worms (DE)
(72) Erfinder: CUCU, Bayram, 65582 Diez (DE); GOUNALAKIS, Charilaos, 67067 Ludwigshafen am Rhein (DE); BLAKAJ, Petrit, 68542 Heddesheim (DE)
(74) Vertreter: Reiser & Partner Patentanwälte mbB
(86) Internationale Anmeldenummer: PCT/EP2024/058379
(87) Internationale Veröffentlichungsnummer: WO 2025/201644

(56) Entgegenhaltungen:
- JP-A- 2003 169 694
- JP-A- H10 236 914
- US-A- 5 827 680
- BURKE VICTOR: "NOTES ON THE GRAM STAIN WITH DESCRIPTION OF A NEW METHOD", JOURNAL OF BACTERIOLOGY, vol. 7, no. 2, 1 March 1922 (1922-03-01), US, pages 159 - 182, XP093205133, ISSN: 0021-9193, Retrieved from the Internet <URL:https://journals.asm.org/doi/pdf/10.1128/jb.7.2.159-182.1922> DOI: 10.1128/jb.7.2.159-182.1922
- N. KOPELOFF & P. BEERMAN: "Modified gram stains", 30 November 1922 (1922-11-30), XP093205331, Retrieved from the Internet <URL:https://www.jstor.org/stable/pdf/30082256.pdf> [retrieved on 20240916]
- SMITH ANN C ET AL: "Gram Stain Protocols", 30 September 2005 (2005-09-30), XP093205322, Retrieved from the Internet <URL:https://asm.org/getattachment/5c95a063-326b-4b2f-98ce-001de9a5ece3/gram-stain-protocol-2886.pdf>

## Beschreibung

Die Gram-Färbung ist eine grundlegende Labortechnik, die zur Unterscheidung und Klassifizierung von Mikroorganismen, insbesondere von Bakterien in die Kategorien Gram-positiv und Gram-negativ verwendet wird. Diese Färbemethode wurde 1884 von dem dänischen Bakteriologen Hans Christian Gram entwickelt und beruht auf der Fähigkeit bakterieller Zellwände, einen violetten Farbstoff während einer Reihe von Färbeschritten zu speichern oder zu verlieren. Grampositive Bakterien behalten den Farbstoff aufgrund ihrer dicken Peptidoglykanschicht und erscheinen unter dem Mikroskop violett bis dunkelblau, während gramnegative Bakterien den Farbstoff aufgrund der besonderen Architektur der Zellhülle mit einer äußeren Membran und nur einer dünnen Peptidoglykanschicht verlieren und bei einer Gegenfärbung rosa bis pink erscheinen. Die Gram-Färbung ist ein unverzichtbares Werkzeug in der Mikrobiologie, welches bei der Identifizierung und Klassifizierung von Mikroorganismen, insbesondere von Bakterien und bei der Steuerung von Behandlungsentscheidungen im klinischen Umfeld hilft.

Die Gram-Färbemethode umfasst sowohl qualitative als auch quantitative Aspekte, wobei zahlreiche Faktoren das Endergebnis beeinflussen, darunter Probendicke, Waschmethoden, Farbstoffkonzentrationen und Entfärbungstechniken. Trotz der Variabilität, die durch diese Faktoren entsteht, bleibt das Verfahren widerstandsfähig und ermöglicht eine gewisse Flexibilität bei den Techniken, während die Präzision innerhalb definierter Grenzen beibehalten wird.

Heutzutage gilt die Gram-Färbemethode weithin als bedeutender Beitrag für die Klinische Diagnostik und Forschung. In der Bakteriologie stellt sie den ersten und äußerst wertvollen Schritt bei der Diagnose und Klassifizierung dar. Die Fähigkeit, den Primärfarbstoff in der Gram-Färbung zu binden, ist eine einzigartige Eigenschaft, die nur bei einem kleinen Teil der natürlichen Materialien auftritt. Die meisten pflanzlichen und tierischen Zellen zeigen eine gramnegative Färbung. Die grampositive Eigenschaft ist hauptsächlich bei Hefen, Bakterien und Schimmelpilzen zu finden. Einige andere Materialien wie Zellkerne, bestimmte Protozoen, Keratohyalin oder Virusproteine wurden als schwach bis stark grampositiv identifiziert.

Bei der Gram-Färbung in ihrer üblichen Ausführung wird nach Anfärbung der Mikroorganismen, insbesondere der Bakterien mit einem basischen Anilinfarbstoff (in der Regel Kristallviolett) durch Nachbehandlung mit Lugolscher Lösung (diese enthält einen Iod-Kaliumiodid-Komplex) ein Farbstoff-Iod-Komplex in den Bakterien gebildet. Dieser Farbkomplex ist im Gegensatz zum primären Farbstoff wasserunlöslich. In Ethanol hingegen ist er löslich und wird deshalb aus gramnegativen Bakterien durch Behandlung mit Ethanol extrahiert. Wegen der dickeren Peptidoglykanschicht wird er dagegen aus grampositiven Bakterien unter den Bedingungen der Gramfärbung durch Ethanol nicht extrahiert.

Der Färbevorgang besteht nach Stand der Technik aus drei Schritten:

### 1. Färben:

Im ersten Schritt färbt man mit einer Lösung von Kristallviolett (Gentianaviolett), der 15 g/l Phenol (Karbolsäure) zugesetzt ist, dem sogenannten "Karbol-Gentianaviolett". Hierbei werden alle Mikroorganismen/Bakterien, grampositive, wie gramnegative, gefärbt. Bei der nachfolgenden Behandlung mit Lugolscher Lösung werden größere Farbstoff-Komplexe gebildet, alle Mikroorganismen/Bakterien erscheinen dunkelblau.

### 2. Entfärben ("Differenzieren"):

Im zweiten Schritt folgt eine Behandlung mit 96%igem Ethanol. Dabei verhalten sich grampositive und gramnegative Mikroorganismen/Bakterien verschieden: gramnegative Mikroorganismen/Bakterien werden schnell wieder entfärbt, während die blauen Farbstoffkomplexe aus grampositiven Mikroorganismen/Bakterien erst nach deutlich längerem Behandeln mit Ethanol ausgewaschen werden können.

### 3. Gegenfärben:

Zur Darstellung der gramnegativen Mikroorganismen/Bakterien können diese abschließend mit verdünnter Fuchsinlösung oder Safraninlösung gegengefärbt werden, worauf sie rot beziehungsweise rotorange erscheinen.

Nach dem Stand der Technik sind die Behandlungen mit Lugolscher Lösung und mit Alkohol die entscheidenden Schritte bei der Gramfärbung.

Nach Stand der Technik werden bei der Gram-Färbe-Methode mehrere Reagenzien verwendet, die potenzielle Gefahrenstoffe darstellen können. Diese Reagenzien umfassen Kristallviolett, Lugolsche Lösung (Iod-Kaliumiodid), Ethanol oder Aceton sowie Safranin. Zu den möglichen Gefahren gehören Hautreizungen, Reizungen der Augen und Atemwege sowie die Entzündbarkeit von Lösungsmittel wie Ethanol oder Aceton, die in den Färbereagenzien enthalten sind. Es ist von entscheidender Bedeutung, beim Umgang mit diesen Reagenzien die entsprechenden Sicherheitsmaßnahmen zu beachten, wie das Tragen persönlicher Schutzausrüstung wie Handschuhe und Schutzbrille sowie die Durchführung der Arbeit in gut belüfteten Räumen.

WO 2001/088177 lehrt eine Färbelösung für Bakterien, die kein Phenol enthält. Die Färbelösung enthält an Stelle von Phenol 4-Methoxyphenol, 2-Phenylethanol, Benzylalkohol, 2-Butoxyethylacetat oder Cyclohexanol.

BURKE, VICTOR, "NOTES ON THE GRAM STAIN WITH DESCRIPTION OF A NEW METHOD" , JOURNAL OF BACTERIOLOGY, Bd. 7, Nr. 2, 1. März 1922 offenbart ein Gram-Färbeverfahren, bei dem Kristallviolett als Primärfärbemittel, eine Lugol-Lösung als Beizlösung, eine Entfärbelösung, die keine Alkohole enthält, und Safranin als Gegenfärbemittel verwendet werden. Darüber hinaus werden alle diese Reagenzien in Form einer wässrigen Lösung verwendet, und die Probe wird nach jeder Reagenzienzugabe mit destilliertem Wasser gewaschen.

N. Kopeloff & P. Beerman, "Modified gram stains ", J . Infectious Diseases, 30. November 1922, beschreibt ein ähnliches Gram-Färbeverfahren unter Verwendung von Fuchsin als Gegenfärbemittel. Darüber hinaus wird bei dem Verfahren die Probe nach jeder Reagenzzugabe nicht mit Wasser gespült.

US 5 827 680 A offenbart eine Variante der ursprünglichen Gram-Färbemethode, die die Gegenfärbung (eine Kombination aus Safranin und Fuchsin) in der Entfärbelösung (eine Mischung aus Aceton und Isopropanol) beinhaltet. Die Farbstoffe werden in Ethanol gelöst.

JP 2003 169694 A offenbart eine Variante der klassischen Gram-Färbemethode unter Verwendung von Entfärbelösungen, die Alkanole enthalten.

JP H10 236914 A offenbart eine Färbezusammensetzung, die Fuchsin (oder Safranin) enthält, das in Diethylenglykolmonoethylether gelöst ist.

Die dieser Erfindung zugrunde liegende Aufgabe bestand darin, ein Verfahren zur Gram-Färbung bereit zu stellen, welches ohne Alkanole, insbesondere Methanol, Ethanol und Isopropanol als Lösemittel auskommt und dadurch die Entzündungsgefahr vermindert.

Die Entwicklung alkanolfreier Reagenzien für die Gram-Färbe-Methode bietet erhebliche Vorteile hinsichtlich der Sicherheit für Personen und der umweltfreundlichen Handhabung bei der Anwendung dieser Färbetechnik in der Mikrobiologie.

Gegenstand der Erfindung ist ein Verfahren zur Einfärbung von Mikroorganismen, insbesondere von Bakterien, umfassend die Schritte
a) Färben einer Bakterienprobe mit einer Lösung von Kristallviolett,
b) Zugabe von Lugolscher Lösung zur gefärbten Bakterienprobe,
c) Spülen mit destilliertem Wasser,
d) Zugabe einer Lösung von Safranin-O und Fuchsin in Diethylenglykolmonoethylether,
e) Spülen mit destilliertem Wasser
wobei das Verfahren im Wesentlichen alkanolfrei durchgeführt wird.

Die für die Gram-Färbung verwendeten Reagenzien sind im Wesentlichen alkanolfrei, insbesondere im Wesentlichen ethanolfrei. Der Begriff "im Wesentlichen alkanolfrei" bedeutet vorliegend, dass der Alkanolgehalt der Lösungen von Kristallviolett und Safranin, sowie der Lugolschen Lösung unter 1 Vol.-%, vorzugsweise unter 0,1 Vol.-% liegt. Gleichfalls liegt der Alkanolgehalt gegebenenfalls zu verwendender Spülflüssigkeiten unter 1 Vol.-%, vorzugsweise unter 0,1 Vol.-%. Während der Durchführung des Verfahrens liegt die Konzentration an Alkanol zu jedem Zeitpunkt unter 1 Vol.-%, vorzugsweise unter 0,1 Vol.-%. Insbesondere steht Alkanol für Methanol, Ethanol und Isopropanol.

Schüttdichten für Färbepigmente, also beispielsweise Kristallviolett, Safranin-O oder Fuchsin, liegen allgemein im Bereich von 0,7 bis 0,8 g/cm³, vorzugsweise bei 0,75 g/cm³. Eine Umrechnung von Volumen- in Gewichtsprozente erfolgt für die Zwecke dieser Erfindung mit einer Schüttdichte von 0,75 g/cm³. Die Schüttdichte von Ammoniumoxalat liegt bei 0,48 g/cm³. Die Schüttdichte von Kaliumiodid liegt bei 1,5 g/cm³.

Angaben in Vol.-%, insbesondere die Volumina der Reagenzien 1, 2 und 3 (siehe Tabellen 1, 2 und 3), beziehen sich auf das gesamte Volumen der betroffenen Zusammensetzung als 100 Vol.-%. Vol.-%-Angaben für das Verfahren beziehen sich auf das gesamte Flüssigkeitsvolumen im jeweils gegebenen Verfahrensschritt.

Neben der verminderten Entzündungsgefahr liegt ein weiterer Vorteil der Erfindung darin, dass ein separater Entfärbeschritt, der im Stand der Technik zumeist mit reinem Ethanol durchgeführt wird, entfällt. Bei dem erfindungsgemäßen Verfahren erfolgt zunächst eine Färbung mit einer Mischung aus Kristallviolett und Lugolscher Lösung in den Schritten a) und b), und danach eine kombinierte Ent- und Gegenfärbung mit einer Safranin/Fuchsin-Lösung in Schritt d).

Die Lösung von Kristallviolett (auch Reagenz 1 genannt), mit der in Schritt a die Bakterienprobe gefärbt wird, enthält neben Kristallviolett (C.I. 42555) ein Lösemittel, vorzugsweise Wasser und Dimethylsulfoxid (DMSO). Daneben enthält diese Lösung ein pH-Stellmittel, vorzugsweise Ammoniumoxalat und Natriumhydrogencarbonat. Die bevorzugte Zusammensetzung von Reagenz 1 zeigt Tabelle 1.

**Tabelle 1: Bevorzugte Zusammensetzung der Reagenz 1 für die Gram-Färbemethode**

| **Reagenz 1: Kristallviolett-Lösung** | | | | |
|---|---|---|---|---|
| | | min. Reinheit | Beispielhafter Anteil (Vol.-%) | Möglicher Anteil (Vol.-%) |
| Bestandteil A | DMSO | ≥ 87 % | 20 | 20 - 70 |
| Bestandteil B | Di-Ammoniumoxalatmonohydrat | ≥ 99 % | 10 | 1 - 10 |
| Bestandteil C | Natrium hydrogencarbonat | ≥ 99 % | 12,5 | 1 - 12,5 |
| Bestandteil D | Kristallviolett (C.I.: 42555) | ≥ 90 % | 10 | 1 - 20 |
| Bestandteil E | dest. Wasser | dest. H₂O | 47,5 | 30 - 77 |

In Tabelle 1 gibt die Spalte "Möglicher Anteil" die allgemeine Zusammensetzung von Reagenz 1 wieder. Die trifft auf die Einträge in den folgenden Tabellen in entsprechender Weise zu.

Die Lugolsche Lösung, oder Reagenz 2, mit der die gefärbte Bakterienprobe in Schritt b) behandelt wird, hat vorzugsweise die in Tabelle 2 angegebene Zusammensetzung, die dem Stand der Technik entspricht.

**Tabelle 2: Zusammensetzung von Reagenz 2 für die Gram-Färbemethode.**

| **Reagenz 2: Lugolsche Lösung** | | | | |
|---|---|---|---|---|
| | | min. Reinheit | Beispielhafter Anteil (Vol.-%) | Möglicher Anteil (Vol.-%) |
| Bestandteil A | Kaliumiodid | ≥ 99 % | 10 | 1 - 30 |
| Bestandteil B | Poly(vinylpyrrolidon)-Iod-Komplex | - | 25 | 1 - 30 |
| Bestandteil C | dest. Wasser | dest. H₂O | 65 | 40 - 98 |

Im Schritt d erfolgt eine gleichzeitige Entfärbung und Gegenfärbung. Die Entfärbung erfolgt durch den Bestandteil A aus dem Reagenz 3, also durch Diethylenglykolmonoethylether. Die Gegenfärbung erfolgt mit den Bestandteilen B und C (Safranin-O und Fuchsin). Reagenz 3 ist eine Lösung von Safranin-O und Fuchsin in Diethylenglykolmonoethylether. Die bevorzugte Zusammensetzung von Reagenz 3 zeigt Tabelle 3.

**Tabelle 3: Zusammensetzung der Reagenz 3 für die Gram-Färbemethode.**

| **Reagenz 3: Entfärber-/Gegenfärber- Lösung** | | | | |
|---|---|---|---|---|
| | | min. Reinheit | Beispielhafter Anteil (Vol.-%) | Möglicher Anteil (Vol.-%) |
| Bestandteil A | Diethylenglykolmonoethylether | ≥ 78 % | 97,54 | 77,5 - 99,4 |
| Bestandteil B | Safranin-O (C.I.: 50240) | ≥ 80 % | 2,1 | 0,5 - 10 |
| Bestandteil C | Basic Fuchsin | ≥ 80 % | 0,36 | 0,1 - 12,5 |

Zwischen den Färbeschritten b) und d) (wie in Tabelle 4 angegeben) erfolgt ein Spülen mit destilliertem Wasser, um überschüssiges Färbereagenz abzuführen. Insbesondere bei der Färbung mit Kristallviolett, ist es erforderlich, nach der vorgesehenen Inkubationszeit eine Spülung durchzuführen, um eine Schädigung der Bakterien durch übermäßige Inkubation zu verhindern.

Im erfindungsgemäßen Verfahren werden Färbeschritte a), b) und d), sowie die Spülschritte c) und e) durchgeführt (wie in Tabelle 4 angegeben). Im ersten Schritt wird die Probe mit Kristallviolett gefärbt, im zweiten Schritt mit der Lugolschen Lösung stabilisiert und anschließend mit destilliertem Wasser kurz gespült. Im Schritt d) wird die gefärbte Probe mit einer Lösung, die Safranin-O und Fuchsin enthält, gleichzeitig entfärbt und gegengefärbt. Nach der Gegenfärbung müssen die Proben wieder mit destilliertem Wasser gespült werden.

Die erfindungsgemäße Färbemethode läuft in einer genauen Reihenfolge (1. - 5.) mit vorgegebenen Inkubationszeiten ab, damit ihre Funktionalität gewährleistet wird. Tabelle 4 zeigt die Primärschritte der Gram-Färbemethode (dabei handelt es sich um die Färbeschritte a), b) und d) sowie das Spülen c) und e).

**Tabelle 4: Schritte der Gram-Färbemethode und Reagenzien**

| | **Schritte** | **Bezeichnung der Reagenzien** |
|---|---|---|
| 1. | a): Primärfärbung | Kristallviolett-Lösung |
| 2. | b): Stabilisierung/Beizung | Lugolsche Lösung |
| 3. | c): Spülen | destilliertes Wasser |
| 4. | d): Ent-/Gegenfärbung | Safranin/Fuchsin Lösung |
| 5. | e): Spülen | destilliertes Wasser |

Die Inkubationszeiten können erheblich variieren. Inkubationszeiten, die als Basiszeitrahmen (funktionelle Zeit) und vorteilhafte Dauer (empfohlene Zeit) betrachtet werden, sind in Tabelle 5 dargestellt. Zusätzlich gibt es verschiedene Inkubationsbereiche, die die Bandbreite der möglichen Zeiten umfassen, innerhalb derer die Reaktionen mit den Reagenzien erfolgreich durchgeführt werden können.

**Tabelle 5: Inkubationszeiten der Gram-Färbe Methode**

| **Protokoll mit Inkubationszeiten** | | | |
|---|---|---|---|
| **Schritte** | **Reagenz** | **empfohlene Zeit (mm:ss)** | **funktionelle Zeit (mm:ss)** |
| A | Kristallviolett-Inkubation | 03:00 | 00:05 - 10:00 |
| B | Inkubation mit Lugolscher Lösung | 01:00 | 00:05 - 10:00 |
| C | Spülen mit dest. Wasser | 00:05 | 00:05 - 05:00 |
| D | Safranin-Fuchsin-Inkubation | 00:25 | 00:20 - 10:00 |
| E | Spülen mit dest. Wasser | 00:30 | 00:05 - 10:00 |

Im Folgenden wird beschrieben, wie sich die im erfindungsgemäßen Verfahren zu verwendenden Reagenzien zusammensetzen.

### Reagenz 1: Kristallviolett-Lösung

**Tabelle 6: Komponentenbezeichnungen und Abkürzungsverzeichnis für die Herstellung einer Kristallviolett-Lösung**

| **Komponente #** | **Name der Komponente** | **Abkürzung** |
|---|---|---|
| Komponente 1 | Kristallviolett (C.I.: 42555) | CV |
| Komponente 2 | Natrium hydrogencarbonat | SBC |
| Komponente 3 | Di-Ammoniumoxalatmonohydrat | DAOx |
| Komponente 4 | Destilliertes Wasser | DW |
| Komponente 5 | Dimethylsulfoxid | DMSO |

### Reagenz 2: Lugolsche Lösung

**Tabelle 7: Komponentenbezeichnungen und Abkürzungsverzeichnis für Lugolsche Lösung (Reagenz 2)**

| **Komponente #** | **Name der Komponente** | **Abkürzung** |
|---|---|---|
| Komponente 1 | Kaliumiodid | KI |
| Komponente 2 | Destilliertes Wasser | DW |
| Komponente 3 | Polyvinylpyrrolidon-Iod | PVP-I |

### Reagenz 3: Entfärber-/Gegenfärber-Lösung

**Tabelle 8: Komponentenbezeichnungen und Abkürzungsverzeichnis für die Herstellung einer Entfärber-/Gegenfärber-Lösung**

| **Komponente #** | **Name der Komponente** | **Abkürzung** |
|---|---|---|
| Komponente 1 | Safranin-O | SF |
| Komponente 2 | Basic Fuchsin | BF |
| Komponente 3 | Diethylenglykolmonoethylether | DEME |

## Patentansprüche

1. Verfahren zur Einfärbung von Mikroorganismen, umfassend die Schritte
a) Färben einer Mikroorganismenprobe mit einer Lösung von Kristallviolett,
b) Zugabe von Lugolscher Lösung zur gefärbten Bakterienprobe,
c) Spülen mit destilliertem Wasser,
d) Zugabe einer Lösung von Safranin-O und Fuchsin in Diethylenglykolmonoethylether,
e) Spülen mit destilliertem Wasser
wobei die Alkanolkonzentration während der Durchführung des Verfahrens zu jedem Zeitpunkt unter 1 Vol.-%, vorzugsweise unter 0,1 Vol.-%, liegt .

2. Verfahren nach Anspruch 1, worin die Lösung von Kristallviolett als Lösemittel Wasser, Dimethylsulfoxid oder eine Mischung daraus enthält.

3. Verfahren nach Anspruch 1 und/oder 2, worin die Lösung von Kristallviolett als pH-Stellmittel Ammoniumoxalat, Natriumhydrogencarbonat oder beides enthält.

4. Verfahren nach Anspruch 2, worin
die Menge an Dimethylsulfoxid 20 - 70 Vol.-%, und
die Menge an Wasser 30 - 77 Vol.-% umfasst.

5. Verfahren nach Anspruch 3, worin
die Menge an Ammoniumoxalat 1 - 10 Vol.-%,
die Menge an Natriumhydrogencarbonat 1 - 12,5 Vol.-%, und
die Menge an Kristallviolett 1 - 20 Vol.-% umfasst.

6. Verfahren nach einem oder mehreren der Ansprüche 1 bis 5, worin die Lugolsche Lösung Wasser, Kaliumiodid und einen Polyvinylpyrrolidon-Iod-Komplex enthält.

7. Verfahren nach Anspruch 6, worin
die Menge an Wasser 40 - 98 Vol.-%
die Menge an Kaliumiodid 1 - 30 Vol.-% und
die Menge an Polyvinylpyrrolidon-Iod-Komplex 1 - 30 Vol.-% beträgt.

8. Verfahren nach einem oder mehreren der Ansprüche 1 bis 7, worin die Lösung von Safranin-O und Fuchsin in Diethylenglykolmonoethylether 77,5 bis 99,4 Vol.-% Diethylenglykolmonoethylether enthält.

9. Verfahren nach einem oder mehreren der Ansprüche 1 bis 7, worin die Lösung von Safranin-O und Fuchsin in Diethylenglykolmonoethylether 0,5 bis 10 Vol.-% Safranin-O oder 0,1 bis 12,5 Vol.-% Fuchsin enthält.

10. Verfahren nach einem oder mehreren der Ansprüche 1 bis 9, worin die Dauer von Schritt a), Färben einer Bakterienprobe mit einer Lösung von Kristallviolett, zwischen 5 s und 10 min beträgt.

11. Verfahren nach einem oder mehreren der Ansprüche 1 bis 10, worin die Dauer von Schritt b), Zugabe von Lugolscher Lösung zur gefärbten Bakterienprobe, zwischen 5 s und 10 min beträgt.

12. Verfahren nach einem oder mehreren der Ansprüche 1 bis 11, worin die Dauer von Schritt c), Spülen mit destilliertem Wasser, zwischen 5 s und 5 min beträgt.

13. Verfahren nach einem oder mehreren der Ansprüche 1 bis 12, worin die Dauer von Schritt d), Zugabe einer Lösung von Safranin-O und Fuchsin in Diethylenglykolmonoethylether, zwischen 20 s und 10 min beträgt.

14. Verfahren nach einem oder mehreren der Ansprüche 1 bis 13, worin die Dauer von Schritt e), Spülen mit destilliertem Wasser, zwischen 5 s und 5 min beträgt.

15. Verfahren nach Anspruch 14, worin Schritt e) einmal wiederholt wird.

## Claims

1. Method for staining microorganisms, comprising the steps of
a) staining a microorganism sample with a solution of crystal violet,
b) adding Lugol's solution to the stained bacterial sample,
c) rinsing with distilled water,
d) adding a solution of safranin-O and fuchsine in diethylene glycol monoethyl ether,
e) rinsing with distilled water,
wherein the alkanol concentration is less than 1% by volume, preferably less than 0.1% by volume, at all times while the method is being carried out.

2. Method according to claim 1, wherein the solution of crystal violet contains water, dimethyl sulphoxide or a mixture thereof as a solvent.

3. Method according to claim 1 and/or claim 2, wherein the solution of crystal violet contains ammonium oxalate, sodium bicarbonate or both as a pH adjusting agent.

4. Method according to claim 2, wherein
the amount of dimethyl sulphoxide is 20-70% by volume, and
the amount of water is 30-77% by volume.

5. Method according to claim 3, wherein
the amount of ammonium oxalate is 1-10% by volume,
the amount of sodium bicarbonate is 1-12.5% by volume, and
the amount of crystal violet is 1-20% by volume.

6. Method according to one or more of claims 1 to 5, wherein the Lugol's solution contains water, potassium iodide and a polyvinylpyrrolidone-iodine complex.

7. Method according to claim 6, wherein
the amount of water is 40-98% by volume,
the amount of potassium iodide is 1-30% by volume, and
the amount of polyvinylpyrrolidone-iodine complex is 1-30% by volume.

8. Method according to one or more of claims 1 to 7, wherein the solution of safranin-O and fuchsine in diethylene glycol monoethyl ether contains 77.5 to 99.4% by volume diethylene glycol monoethyl ether.

9. Method according to one or more of claims 1 to 7, wherein the solution of safranin-O and fuchsine in diethylene glycol monoethyl ether contains 0.5 to 10% by volume safranin-O or 0.1 to 12.5 % by volume fuchsine.

10. Method according to one or more of claims 1 to 9, wherein the duration of step a), staining a bacterial sample with a solution of crystal violet, is between 5 s and 10 min.

11. Method according to one or more of claims 1 to 10, wherein the duration of step b), adding Lugol's solution to the stained bacterial sample, is between 5 s and 10 min.

12. Method according to one or more of claims 1 to 11, wherein the duration of step c), rinsing with distilled water, is between 5 s and 5 min.

13. Method according to one or more of claims 1 to 12, wherein the duration of step d), adding a solution of safranin-O and fuchsine in diethylene glycol monoethyl ether, is between 20 s and 10 min.

14. Method according to one or more of claims 1 to 13, wherein the duration of step e), rinsing with distilled water, is between 5 s and 5 min.

15. Method according to claim 14, wherein step e) is repeated once.

## Revendications

1. Procédé de coloration de microorganismes comprenant les étapes
a) de coloration d'un échantillon de microorganismes avec une solution de violet cristallisé,
b) d'addition d'une solution de Lugol à l'échantillon bactérien coloré,
c) de rinçage avec de l'eau distillée,
d) d'addition d'une solution de safranine O et de fuchsine dans du diéthylène glycol monoéthyl éther,
e) de rinçage avec de l'eau distillée,
dans lequel la concentration en alcanol pendant l'exécution du procédé se situe à chaque instant en-dessous de 1 % en volume, de préférence en-dessous de 0,1 % en volume.

2. Procédé selon la revendication 1, dans lequel la solution de violet cristallisé contient en tant que solvant de l'eau, du diméthylsulfoxyde ou un mélange de ceux-ci.

3. Procédé selon la revendication 1 et/ou 2, dans lequel la solution de violet cristallisé contient en tant qu'agent de réglage du pH de l'oxalate d'ammonium, de l'hydrogénocarbonate de sodium ou les deux.

4. Procédé selon la revendication 2, dans lequel
la quantité de diméthylsulfoxyde comprend 20 à 70 % en volume et
la quantité d'eau comprend 30 à 77 % en volume.

5. Procédé selon la revendication 3, dans lequel
la quantité d'oxalate d'ammonium comprend 1 à 10 % en volume,
la quantité d'hydrogénocarbonate de sodium comprend 1 à 12,5 % en volume, et
la quantité de violet cristallisé comprend 1 à 20 % en volume.

6. Procédé selon une ou plusieurs des revendications 1 à 5, dans lequel la solution de Lugol contient de l'eau, de l'iodure de potassium et un complexe de polyvinyl pyrrolidone et d'iode.

7. Procédé selon la revendication 6, dans lequel
la quantité d'eau est de 40 à 98 % en volume,
la quantité d'iodure de potassium est de 1 à 30 % en volume, et
la quantité de complexe polyvinyl pyrrolidone-iode est de 1 à 30 % en volume.

8. Procédé selon une ou plusieurs des revendications 1 à 7, dans lequel la solution de safranine O et de fuchsine dans le diéthylène glycol monoéthyl éther contient de 77,5 à 99,4 % en volume de diéthylène glycol monoéthyl éther.

9. Procédé selon une ou plusieurs des revendications 1 à 7, dans lequel la solution von safranine O et de fuchsine dans le diéthylène glycol monoéthyl éther contient de 0,5 à 10 % en volume de safranine O ou de 0,1 à 12,5 % en volume de fuchsine.

10. Procédé selon une ou plusieurs des revendications 1 à 9, dans lequel la durée de l'étape a) de coloration d'un échantillon bactérien avec une solution de violet cristallisé est entre 5 s et 10 min.

11. Procédé selon une ou plusieurs des revendications 1 à 10, dans lequel la durée de l'étape b) d'addition de la solution de Lugol à l'échantillon bactérien coloré est entre 5 s et 10 min.

12. Procédé selon une ou plusieurs des revendications 1 à 11, dans lequel la durée de l'étape c) de rinçage avec de l'eau distillée est entre 5 s et 5 min.

13. Procédé selon une ou plusieurs des revendications 1 à 12, dans lequel la durée de l'étape d) d'addition d'une solution de safranine O et de fuchsine dans du diéthylène glycol monoéthyl éther est entre 20 s et 10 min.

14. Procédé selon une ou plusieurs des revendications 1 à 13, dans lequel la durée de l'étape e) de rinçage avec de l'eau distillée est entre 5 s et 5 min.

15. Procédé selon la revendication 14, dans lequel l'étape e) est répétée une fois.
